# EUROPÄISCHE PATENTSCHRIFT

(11) **EP 0 489 097 B1**
(45) Veröffentlichungstag und Bekanntmachung des Hinweises auf die Patenterteilung: **26.07.1995**
(21) Anmeldenummer: 90913317.5
(22) Anmeldetag: 18.08.1990
(51) Int. Cl.: C07C 59/125, C07C 59/13, C07C 59/305, C07C 59/31, C07C 51/235, C07C 41/03

(54) **VERFAHREN ZUR HERSTELLUNG VON ÄTHERCARBONSÄUREN AUS KOHLENHYDRATEN UND DEREN DERIVATEN**
METHOD FOR PREPARING ETHER CARBOXYLIC ACIDS FROM CARBOHYDRATES AND THEIR DERIVATIVES
METHODE DE PREPARATION D'ACIDES CARBOXYLIQUES D'ETHER A PARTIR D'HYDRATES DE CARBONE, LEURS DERIVES

(30) Priorität: 26.08.1989 DE 3928310
(43) Veröffentlichungstag der Anmeldung: 10.06.1992
(60) Teilanmeldung: 95100118.9
(73) Patentinhaber: HOECHST AKTIENGESELLSCHAFT, 65926 Frankfurt am Main (DE)
(72) Erfinder: SCHÖNWÄLDER, Karl-Heinz, D-6233 Kelkheim (DE); LEUPOLD, Ernst, Ingo, D-6392 Neu-Anspach (DE); GOHLA, Werner, D-5216 Niederkassel (DE); DANY, Franz-Josef, D-5042 Erftstadt (DE); SCHLINGMANN, Merten, D-6240 Königstein (DE)
(86) Internationale Anmeldenummer: EP9001364
(87) Internationale Veröffentlichungsnummer: WO9102712

(56) Entgegenhaltungen:
- EP-A- 0 302 007
- FR-A- 2 391 185
- US-A- 3 799 977
- PATENT ABSTRACTS OF JAPAN, Band 6, Nr. 131 (C-114) (1009), 17. Juli 1982; & JP-A-5758642

## Beschreibung

Die vorliegende Erfindung betrifft ein Verfahren zur Herstellung von Äthercarbonsäuren durch Oxäthylierung von Kohlenhydraten und anschließende katalytische Oxidation sowie die Verwendung der erhaltenen Produkte.

Wegen der eutrophierenden Wirkung in Gewässern ist die Verwendung von Phosphaten in Wasch- und Reinigungsmitteln in einer Reihe von Ländern gesetzlich beschränkt, z. T. sogar verboten. Daher ist inzwischen eine Vielzahl von Ersatzstoffen für die Phosphate, insbesondere für das Natriumtripolyphosphat, als Gerüststoff ("Builder") entwickelt und vorgeschlagen worden. Jedoch werden die wünschenswerten waschtechnischen Eigenschaften des Natriumtripolyphosphates bisher von keinem Einzelstoff im ganzen erreicht. Vielmehr sind lediglich Kombinationen von Gerüststoffen in der Lage, in erster Näherung die Wirkung der Phosphate zu erreichen. Nur relativ wenige Ersatzstoffe für Phosphate - oder besser gesagt Teilersatzstoffe - sind hinsichtlich ihrer ökologischen Eigenschaften voll befriedigend. Wenn sie die Eutrophierung der Gewässer auch nicht fördern, so haben sie z. T. aber doch Eigenschaften, die für die Umwelt als bedenklich angesehen werden müssen, wie die Remobilisierung von Schwermetallen aus den Sedimenten der Gewässer oder mangelnde biologische Abbaubarkeit; daher ist ihre Umweltrelevanz, auch wenn diese Stoffe nach heutigem Kenntnisstand zunächst einmal nicht als toxisch einzustufen sind, ungewiß. Die Suche nach effektiven Gerüststoffen für Waschmittel, die bezüglich ihrer ökologischen Wirkung als unbedenklich eingestuft werden können, geht daher weiter.

Aus der japanischen Offenlegungsschrift 58-117284 sind sogenannte Viskositätsverminderer von Steinkohlenschlämmen bekannt, die durch Umsetzung mehrwertiger Alkohole, zu denen auch Kohlenhydrate gerechnet werden, mit Alkylenoxiden zu Polyätherverbindungen sowie gegebenenfalls anschließende Umwandlung der endständigen Hydroxygruppen zu Carboxylgruppen hergestellt werden können; genauere Angaben über die Synthese dieser Produkte werden jedoch nicht gemacht.

Die Oxäthylierung von Kohlenhydraten ist seit längerem bekannt. So hat z. B. W. Gerhardt, J. f. prakt. Chem., 4. Reihe, 29, 300 (1965), die Oxäthylierung von Saccharose beschrieben; in der japanischen Offenlegungsschrift 58-117284 sind noch weitere Kohlenhydrate erwähnt. Jedoch sind wirtschaftliche Verfahren zur Herstellung oxidierter Oxäthylate von Kohlenhydraten ebenso wie die Verwendung solcher Produkte als Gerüststoff bisher nicht bekannt.

Es bestand daher die Aufgabe, biologisch abbaubare Ersatzstoffe für Phosphate sowie eine wirtschaftliche und technisch durchführbare Herstellungsmethode zu entwickeln.

Überraschenderweise wurde nun gefunden, daß man biologisch abbaubare Ersatzstoffe für Phosphate mit gutem Kalkbindevermögen erhält, wenn man Kohlenhydrate oxäthyliert, gegebenenfalls unter zusätzlicher Oxalkylierung durch ein höheres Alkylenoxyd, und die endständigen primären Hydroxylgruppen durch katalytische Oxidation in Carboxylgruppen überführt.

Gegenstand der Erfindung ist somit ein Verfahren zur Herstellung von Äthercarbonsäuren durch Oxalkylierung von Kohlenhydraten und deren Derivaten und anschließende Oxidation, dadurch gekennzeichnet, daß man die Kohlenhydrate oder deren Derivate in üblicher Weise entweder unmittelbar äthoxyliert oder zunächst mit Propylenoxid, Butylenoxid oder Styroloxid alkoxyliert und dann äthoxyliert und die dabei erhaltene wäßrige Lösung ohne weitere Behandlung in einem pH-Bereich, in dem die Kohlenhydrate und davon abgeleitete Carbonsäuren beständig sind, mit Sauerstoff als Oxidationsmittel in Gegenwart eines Katalysators, der wenigstens ein Platinmetall enthält, oxidiert.

Die nach dem erfindungsgemäßen Verfahren hergestellten Äthercarbonsäuren eignen sich wegen ihres überraschend hohen Kalkbindevermögens, insbesondere als Gerüststoffe in Wasch- und Reinigungsmitteln.

Als Ausgangsmaterial sind praktisch alle Kohlenhydrate mit mindestens einer oxalkylierbaren Hydroxygruppe und einer zu einer Carboxylgruppe oxidierbaren Alkoholgruppe geeignet, wie Glucose, Galactose, Maltose, Lactose, Fruktose, β-Cyclodextrin, insbesondere Saccharose. Als Derivate seien beispielsweise genannt α-Methyl-D-glucosid, Sorbit, Mannit, 2-Desoxy-D-ribose und D-Glucosamin.

Die Oxäthylierung und Oxalkylierung erfolgen in üblicher Weise, und zwar im allgemeinen in wäßriger Lösung unter Einwirkung basischer Katalysatoren bei Temperaturen zwischen 50 und 150 °C, vorzugsweise in einem Druckbereich von Atmosphärendruck bis 15 bar. Die Lösung, die die Verbindungen mit den endständigen Hydroxyäthylgruppen enthält, wird, vorzugsweise nach Verdünnung auf einen Wassergehalt von 70 - 90 Gew.-% der katalytischen Oxidation unterworfen, wobei die Äthercarbonsäuren entstehen. Von den Hydroxylgruppen der Kohlenhydrate und der Derivate sollen zweckmäßig mindestens 50 % alkoxyliert sein, vorzugsweise mindestens 75 % und insbesondere etwa 100 %.

Als höhere Alkylenoxide kommen Butylenoxid, Styroloxid und vor allem Propylenoxid in Frage. Diese können z. B. in einer Menge von bis zu 3 Mol, zweckmäßig von mindestens 0,1 Mol verwendet werden. Naturgemäß ist der Anteil der höheren Alkylenoxide zweckmäßig so zu wählen, daß die biologische Abbaubarkeit gewährleistet bleibt.

Das Äthylenoxid wird zweckmäßig in einer Menge von mindestens 1 Mol, z. B. bis zu 10 Mol und vorzugsweise bis zu 5 Mol verwendet. Dabei beziehen sich alle Molangaben auf Gramm-Mol Hydroxylgruppen, die in dem Kohlenhydrat bzw. dessen Derivat enthalten sind. Sofern diese zunächst mit einem anderen Alkylenoxid als Äthylenoxid umgesetzt werden, beträgt die Gesamtmenge des umgesetzten Alkylenoxids, also einschließlich des Äthylenoxids, zweckmäßig nicht mehr als 5 Mol.

Als Katalysatoren eignen sich solche, die Platinmetalle, also Osmium, Iridium, Rhodium, Ruthenium, Palladium und/oder Platin, enthalten. Bevorzugt sind Katalysatoren, die eine Kombination von Palladium und Platin und insbesondere nur Platin enthalten. Vorzugsweise sind die Platinmetalle auf einem Träger, wie Al₂O₃ oder SiO₂, insbesondere auf Aktivkohle aufgebracht. Der Metallgehalt des Katalysators liegt im allgemeinen bei 1 bis 15, vorzugsweise bei 5 bis 10 Gew.-%.

Zuweilen kann es zweckmäßig sein, insbesondere wenn man Kohlenhydratderivate mit schlechterer Wasserlöslichkeit als Ausgangsstoffe verwendet, einen unter den Reaktionsbedingungen inerten Lösungsvermittler, vorzugsweise in einer Konzentration von 10 bis 75 Gew.-%, insbesondere 30 bis 50 Gew.-%, bezogen auf die Menge an Wasser und Lösungsvermittler, zuzusetzen. Geeignet sind vor allem solche Lösungsvermittler, die beim Durchleiten von Sauerstoff durch die wäßrige Lösung wenig flüchtig sind, so daß eine Explosionsgefahr im Dampfraum weitgehend vermieden wird; auf der anderen Seite sollte der Lösungsvermittler nach der Oxydation leicht abtrennbar sein, beispielsweise durch Destillation. Geeignete Lösungsvermittler sind beispielsweise Glykoläther ohne freie OH-Gruppen, wie solche der Formel R¹O(CHRCH₂O)ₙR², wobei n eine Zahl von 1 bis 4, R H oder CH₃ und R¹ und R² jeweils unabhängig voneinander C₁-C₄-Alkyl bedeuten. Besonders geeignet sind die Dimethyl-, Diäthyl- und Methyl-äthyl-äther der genannten allgemeinen Formel mit Siedepunkten im Bereich von 100 bis etwa 250°C, beispielsweise Triäthylenglykoldimethyläther und bevorzugt Diäthylenglykoldimethyläther.

Bevorzugtes Oxidationsmittel ist reiner Sauerstoff. Es können jedoch auch Mischungen von Sauerstoff mit unter den Reaktionsbedingungen inerten Gasen, beispielsweise Mischungen von Sauerstoff mit Inertgasen, verwendet werden. Natürlich ist auch Luft selbst geeignet.

In der Regel arbeitet man bei der Oxidation bei einem Gesamtdruck von 0,5 bis 100 bar. Die Reaktionsgeschwindigkeit steigt mit steigendem Sauerstoffpartialdruck deutlich an; jedoch kann der Vorteil der höheren Reaktionsgeschwindigkeit durch den bei Anwendung von höherem Druck erforderlichen höheren apparativen Aufwand in Bezug auf die Wirtschaftlichkeit überkompensiert werden. Bevorzugt ist ein Druckbereich von Atmosphärendruck bis 10 bar (absolut), wobei das Arbeiten bei Atmosphärendruck besonders einfach auszuführen ist.

Die Oxidation wird in der Regel bei einer Temperatur von 5 bis 80°C, vorzugsweise von 10 bis 60°C, insbesondere von 20 bis 40°C durchgeführt. Da viele Kohlenhydrate und davon abgeleitete Carbonsäuren im sauren Bereich wenig beständig sind, wird die Oxidation zweckmäßig im etwa neutralen bis schwach alkalischen Medium, also im pH-Bereich von 5 bis 9, vorzugsweise von 6 bis 8,5 und insbesondere von 7 bis 8, durchgeführt. Die während der Oxydation entstehenden Carbonsäuren werden zweckmäßig abgefangen, z.B. durch geeignete Puffersubstanzen oder vorteilhaft durch Zugabe von wäßrigen Basen, etwa Alkali- oder Erdalkalihydroxyd-Lösungen, wobei diese zweckmäßig so zudosiert werden, daß der pH-Wert des Reaktionssystems während der Oxydation im Bereich von 6 bis 9 bleibt. Bei einer vollständigen Neutralisation fallen die Oxydationsprodukte dann als Salze an.

Das erfindungsgemäße Verfahren kann in allen Apparaturen, die sich für die Durchführung von Reaktionen in der flüssigen Phase mit oder ohne Anwendung von Überdruck eignen, durchgeführt werden. Beispiele dafür sind die Durchführung in einem Rührkessel oder in einer Blasensäule mit suspendiertem Katalysator. Die Oxydation kann aber auch an einem Festbett mit gekörntem Katalysator in einem Rieselphasenreaktor durchgeführt werden.

Die erforderliche Reaktionszeit wird zweckmäßig dadurch ermittelt, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeitschromatographie im Vergleich zu Standardlösungen laufend bestimmt werden. Die Optimierung der Reaktionszeit ist zu empfehlen, da eine unnötig verlängerte Einleitung von Sauerstoff zu Überoxydationen und damit beispielsweise zu Decarboxylierungen und zur Verminderung der Ausbeute an den gewünschten Reaktionsprodukten führen kann.

Das Reaktionsgemisch kann nach üblichen Methoden aufgearbeitet werden. Z.B. werden zunächst das Wasser und etwa vorhandene Lösungsvermittler destillativ entfernt. Anschließend wird eine Reinigung, z.B. durch Chromatographie, Kristallisation oder Fällung, vorgenommen. Auch eine Abtrennung aus der bei der Oxydation erhaltenen Lösung über basische Ionenaustauscher in der OH⁻-Form ist möglich. Im allgemeinen hat es sich jedoch als vorteilhaft erwiesen, die bei der Reaktion erhaltene Lösung einer Sprühtrocknung zu unterwerfen.

Das Kalkbindevermögen der Äthercarbonsäuren kann durch übliche Analysenmethoden ermittelt werden, beispielsweise durch Trübungstitration bzw. potentiometrische Titration mittels einer ionenspezifischen Elektrode.

Die erfindungsgemäß erhältlichen Äthercarbonsäuren eignen sich vor allem als Zusatzstoff (Gerüststoff) in Wasch- oder Reinigungsmitteln. Daneben sind sie auch als Lebensmittelzusatzstoff, als Vernetzer in Lackzubereitungen udgl. einsetzbar.

Die nachfolgenden Beispiele erläutern die Erfindung.

### Beispiel 1 - Herstellung

In einem 2 l-Rührautoklaven wurden 111 g Saccharose und 2,75 g KOH in 450 ml Wasser mit 333 g Äthylenoxid so versetzt, daß die Temperatur zwischen 85 und 100 °C lag und der Druck bis zu 4 bar betrug. Nach 2 Stunden Reaktionszeit wurde die Lösung auf Raumtemperatur abgekühlt und mit Wasser auf 4 l Gesamtvolumen aufgefüllt. Diese Lösung wurde in einem von außen beheizten senkrecht angeordneten Glasrohr (Durchmesser 100 mm, Länge 800 mm) nach Zugabe von 20 g eines handelsüblichen Katalysators (5 % Platin auf Aktivkohle) bei 50 °C von unten durch eine Glasfritte mit 100 NL/h Sauerstoff begast. Durch kontinuierliche Zugabe von 30 %iger wäßriger Natronlauge wurde der pH-Wert bei 7 bis 7,5 gehalten. Die Oxidationszeit betrug 6 h. Durch Sprühtrocknung des Reaktionsproduktes erhielt man 390 g eines farblosen Pulvers mit einem Kalkbindevermögen von 279 mg CaCO₃ pro g Substanz (ermittelt bei Raumtemperatur).

### Beispiel 2 - Herstellung

Analog Beispiel 1 wurden 110 g Sorbit mit 470 g Ethylenoxid und 4 g KOH in 950 ml Wasser ethoxyliert und anschließend nach Zugabe von 4,27 l Wasser und 290 g Katalysator oxidiert. Das sprühgetrocknete Produkt (484 g) hatte ein Kalkbindevermögen von 373 mg CaCO₃/g Substanz.

### Beispiel 3 - Herstellung

Analog Beispiel 1 wurden Pentaerythrit und α-Methylglucosid ethoxyliert und oxidiert und nach Aufarbeitung hinsichtlich ihres Kalkbindevermögens untersucht. Das mit Pentaerythrit erhaltene Produkt besaß ein Kalkbindevermögen von 343 mg CaCO₃/g Substanz und das mit α-Methylglucosid ein solches von 327 mg CaCO₃/g Substanz.

## Patentansprüche

1. Verfahren zur Herstellung von Äthercarbonsäuren durch Oxalkylierung von Kohlenhydraten und deren Derivaten und anschließende Oxidation, dadurch gekennzeichnet, daß man die Kohlenhydrate oder deren Derivate in üblicher Weise entweder unmittelbar äthoxyliert oder zunächst mit Propylenoxid, Butylenoxid oder Styroloxid alkoxyliert und dann äthoxyliert und die dabei erhaltene wäßrige Lösung ohne weitere Behandlung in einem pH-Bereich, in dem die Kohlenhydrate und davon abgeleitete Carbonsäuren beständig sind, mit Sauerstoff als Oxidationsmittel in Gegenwart eines Katalysators, der wenigstens ein Platinmetall enthält, oxidiert.

2. Verfahren nach Anspruch 1, dadurch gekennzeichnet, daß die Menge des höheren Alkylenoxids bis zu 3 Mol und die des Äthylenoxids von 1 bis 10 Mol, jeweils bezogen auf 1 Gramm-Mol OH in dem Kohlenhydrat und dem Derivat davon, beträgt, wobei die Gesamtmenge an Alkylenoxiden vorzugsweise bis zu 5 Mol beträgt.

3. Verfahren nach Anspruch 1 oder 2, dadurch gekennzeichnet, daß der Katalysator als Platinmetall eine Kombination von Palladium und Platin, insbesondere nur Platin enthält.

4. Verfahren nach einem oder mehreren der Ansprüche 1 bis 3, dadurch gekennzeichnet, daß der Katalysator aus 1 bis 15 Gew.-%, vorzugsweise 5 bis 10 Gew.-%, des Platinmetalls und einem Träger, vorzugsweise Aktivkohle, besteht.

5. Verfahren nach einem oder mehreren der Ansprüche 1 bis 4, dadurch gekennzeichnet, daß die Oxydation in einem Druckbereich von 0,5 bis 100 bar, vorzugsweise von Atmosphärendruck bis 10 bar, insbesondere bei Atmosphärendruck, durchgeführt wird.

6. Verfahren nach einem oder mehreren der Ansprüche 1 bis 5, dadurch gekennzeichnet, daß die wäßrige Lösung einen unter den Reaktionsbedingungen inerten Lösungsvermittler, vorzugsweise in einer Menge von 10 bis 75 Gew.-%, insbesondere 30 bis 50 Gew.-%, enthält, wobei der Lösungsvermittler vorzugsweise ein Glykoläther ohne Hydroxygruppen, insbesondere Diäthylenglykoldimethyläther ist.

7. Verfahren nach einem oder mehreren der Ansprüche 1 bis 6, dadurch gekennzeichnet, daß die Oxydation bei einer Temperatur von 5 bis 80°C, vorzugsweise von 10 bis 60°C, insbesondere von 20 bis 40°C, durchgeführt wird.

8. Verfahren nach einem oder mehreren der Ansprüche 1 bis 7, dadurch gekennzeichnet, daß die Oxydation bei einem pH-Wert von 5 bis 9, vorzugsweise von 6 bis 8,5 und insbesondere von 7 bis 8 durchgeführt wird.

## Claims

1. A process for the preparation of ether carboxylic acids by alkoxylation of carbohydrates and derivatives thereof, followed by oxidation, which comprises either ethoxylating the carbohydrates or derivatives thereof directly in the usual manner or first alkoxylating them using propylene oxide, butylene oxide or styrene oxide and then ethoxylating them and oxidizing the aqueous solution thus obtained without any further treatment in a pH range in which the carbohydrates and carboxylic acids derived therefrom are stable, using oxygen as the oxidizing agent in the presence of a catalyst containing at least one platinum metal.

2. The process as claimed in claim 1, wherein the amount of the higher alkylene oxide is up to 3 mol and that of the ethylene oxide is from 1 to 10 mol, in each case relative to 1 mol of OH in the carbohydrate and the derivative thereof, the total amount of alkylene oxides being preferably up to 5 mol.

3. The process as claimed in claim 1 or 2, wherein the catalyst contains a combination of palladium and platinum, in particular only platinum, as the platinum metal.

4. The process as claimed in one or more of claims 1 to 3, wherein the catalyst comprises 1 to 15% by weight, preferably 5 to 10% by weight, of the platinum metal and a support, preferably activated carbon.

5. The process as claimed in one or more of claims 1 to 4, wherein the oxidation is carried out in a pressure range from 0.5 to 100 bar, preferably from atmospheric pressure to 10 bar, in particular at atmospheric pressure.

6. The process as claimed in one or more of claims 1 to 5, wherein the aqueous solution contains a solubilizing agent which is inert under the reaction conditions, preferably in an amount of 10 to 75% by weight, in particular 30 to 50% by weight, the solubilizing agent being preferably a glycol ether without hydroxyl groups, in particular diethylene glycol dimethyl ether.

7. The process as claimed in one or more of claims 1 to 6, wherein the oxidation is carried out at a temperature from 5 to 80°C, preferably from 10 to 60°C, in particular from 20 to 40°C.

8. The process as claimed in one or more of claims 1 to 7, wherein the oxidation is carried out at a pH from 5 to 9, preferably from 6 to 8.5 and in particular from 7 to 8.

## Revendications

1. Procédé de fabrication d'acides éthers-carboxyliques par oxalkylation de glucides ou de dérivés de ceux-ci, suivie d'une oxydation, procédé caractérisé en ce que, en opérant de la manière courante, ou bien on éthoxyle directement les glucides ou leurs dérivés, ou bien on les alcoxyle d'abord avec de l'oxyde de propylène, de butylène ou de styrène, puis on effectue l'éthoxylation, et on soumet la solution aqueuse ainsi obtenue, sans autre traitement et dans un intervalle de pH dans lequel les glucides et les acides carboxyliques qui en proviennent restent stables, à une oxydation avec de l'oxygène comme oxydant en présence d'un catalyseur comprenant au moins un métal de la famille du platine.

2. Procédé selon la revendication 1, caractérisé en ce que la proportion de l'oxyde d'alkylène supérieur s'élève jusqu'à 3 moles et celle de l'oxyde d'éthylène est de 1 à 10 moles, chacune rapportée à une gramme-mole de OH dans le glucide ou dans son dérivé, la proportion totale des oxydes d'alkylènes allant de préférence jusqu'à 5 moles.

3. Procédé selon la revendication 1 ou 2, caractérisé en ce que le catalyseur comprend, comme métal de la famille du platine, une combinaison de palladium et de platine ou en particulier seulement du platine.

4. Procédé selon une ou plusieurs des revendications 1 à 3, caractérisé en ce que le catalyseur est formé de 1 à 15 % en poids, de préférence de 5 à 10 %_{,} du métal de la famille du platine et d'un support, de préférence du charbon actif.

5. Procédé selon une ou plusieurs des revendications 1 à 4, caractérisé en ce que l'on effectue l'oxydation dans un intervalle de pressions de 0,5 à 100 bar, de préférence compris entre la pression atmosphérique et 10 bar, et plus spécialement à la pression atmosphérique.

6. Procédé selon une ou plusieurs des revendications 1 à 5, caractérisé en ce que la solution aqueuse contient un adjuvant de dissolution inerte dans les conditions réactionnelles, de préférence dans une proportion de 10 à 75 % en poids et plus spécialement de 30 à 50 %, adjuvant qui est avantageusement un éther de glycol sans groupes hydroxyliques, en particulier l'éther diméthylique du diéthylène-glycol.

7. Procédé selon une ou plusieurs des revendications 1 à 6, caractérisé en ce que l'on effectue l'oxydation à une température de 5 à 80°C, de préférence de 10 à 60°C, et plus spécialement de 20 à 40°C.

8. Procédé selon une ou plusieurs des revendications 1 à 7, caractérisé en ce que l'on effectue l'oxydation à un pH de 5 à 9, de préférence de 6 à 8,5, et plus spécialement de 7 à 8.
